# EUROPEAN PATENT APPLICATION

(11) **EP 0 898 970 A1**
(43) Date of publication of application: **03.03.1999**
(21) Application number: 97904569.7
(22) Date of filing: 19.02.1997
(51) Int. Cl.: A61K 49/00, A61K 51/10, A61K 39/395, G01N 33/534, G01N 33/577

(54) **DIAGNOSTIC AGENT FOR DIGESTIVE DISEASES**

(30) Priority: 19.02.1996 JP 55513/96; 18.03.1996 JP 90035/96
(71) Applicant: YAMASA CORPORATION, Choshi-shi, Chiba 288 (JP)
(72) Inventor: IWASAKI, Tsutomu, Maebashi-shi, Gumma 371 (JP); NAGAI, Ryozo, Maebashi-shi, Gumma 371 (JP); KATOH, Hirohisa, Choshi-shi, Chiba 288 (JP)
(74) Representative: Helbing, Jörg, Dr. Dipl.-Chem.
(86) International application number: JP9700438
(87) International publication number: WO9729785

(57) **Abstract**

Antibody reagent and kit containing an antibody against human smooth muscle myosin or active fragments thereof and being usable for detecting digestive diseases; and methods of screening and diagnostic imaging for these diseases by use of the same. The invention permits emergency testing in the diagnosis of these diseases and the specification of the affected region.

## Description

### Technical Field

The present invention relates to an antibody reagent and kit for screening digestive diseases. The present invention also relates to a diagnostic agent and kit that are useful for diagnostic imaging for digestive diseases.

### Background Art

Ischemic colitis and enterostasis are grave diseases caused by blood circulation disorders of the intestines. The causes of blood circulation disorders of the intestines vary and include thromboembolism attributable to cardiovascular disorders resulting from arteriosclerosis or angiitis in the whole body, as well as local blood circulation disorders caused by strangulated enterostasis, intussusception, volvulus, and the like. Reflecting the recent increase in arteriosclerotic circulatory diseases, intestinal circulation disorders in particular are increasing. Disorders of blood circulation to the intestines result in necrosis in a wide area of intestinal tissue as well as necrosis of intestine smooth muscle cells. Significantly severe cases are characterized by intense abdominal pain, sometimes leading to terebration, and a high mortality rate. Ulcerative colitis, Crohn's disease, and ulcerative digestive diseases caused by radiation, drugs, collagen disease, infectious diseases, malignant neoplasm, and the like may also result in peritoneal terebration, accompanied by necrosis of intestinal smooth muscle cells, when the pathemas become grave. Many of the diseases that damage smooth muscle layers protecting the abdomen at the deepest part of the intestinal wall are grave, and speedy, accurate diagnosis is indispensable for proper treatment of these diseases.

Conventionally, intestine smooth muscle disorders have been diagnosed based on the results of abdominal roentogenologic examination and diagnostic imaging tests such as endoscopy, as well as abnormal values obtained from general biochemical blood tests and attributed to a primary disease, since there exist no test methods specific to these diseases. Thus, diagnosis has been very difficult.

Among these diagnostic methods, roentogenography, echography, CT (X-ray computer tomography), digestive tract contrast test, endoscopy, and vascular contrast test have been used as methods of diagnostic imaging for ischemic digestive diseases and grave ulcerative digestive diseases, similar to other digestive diseases.

However, digestive tract contrast test and a vascular contrast test, which are considered dangerous, are not generally accepted. In particular, digestive tract contrast test, endoscopy, and vascular contrast test have poor ability to diagnose diseases of the small intestine. Furthermore, echography and CT are not useful for diagnosis of diseases of the stomach and intestines.

Therefore, an object of the present invention is to provide an antibody reagent usable for detecting *in vitro* ischemic digestive diseases and grave ulcerative digestive diseases by binding specifically to a region affected by these diseases. Another object of the present invention is to develop a tracer preparation to enable correct diagnostic imaging for an affected region *in vivo* by binding specifically to a region affected by digestive diseases.

### Disclosure of the Invention

The present inventors have conducted studies and have found that smooth muscle myosin is liberated into blood in digestive diseases and that detecting smooth muscle myosin is useful for diagnosing these diseases. The present invention was accomplished based on this finding.

Accordingly, the present invention provides an antibody reagent and kit comprising an antibody against human smooth muscle myosin or active fragments thereof to screen digestive diseases.

The present invention also provides a method for screening digestive diseases through measured values obtained by measuring smooth muscle myosin in a sample.

Further, the present invention provides an image-diagnostic agent and kit for digestive diseases comprising a monoclonal antibody against human smooth muscle myosin or active fragments of the antibody labeled with radioactive isotopes.

Yet further, the present invention provides methods of screening and diagnostic imaging for digestive diseases by using the above diagnostic agent or kit to specify the region affected by digestive diseases.

### Brief Description of Drawings

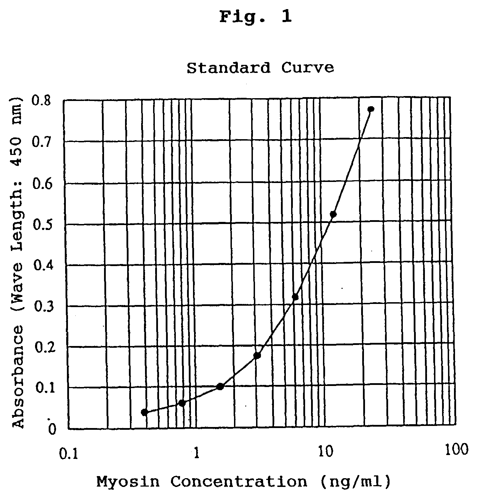

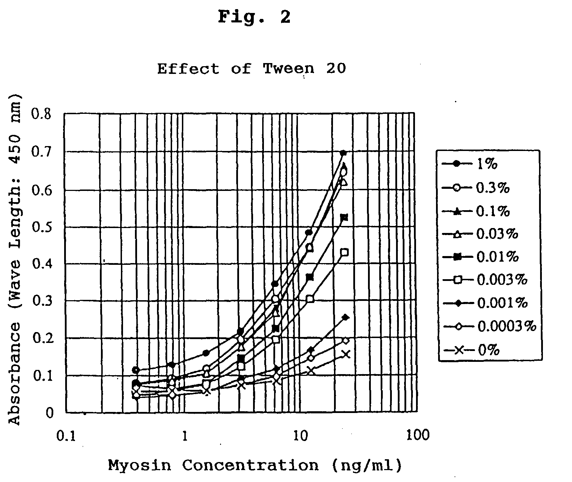

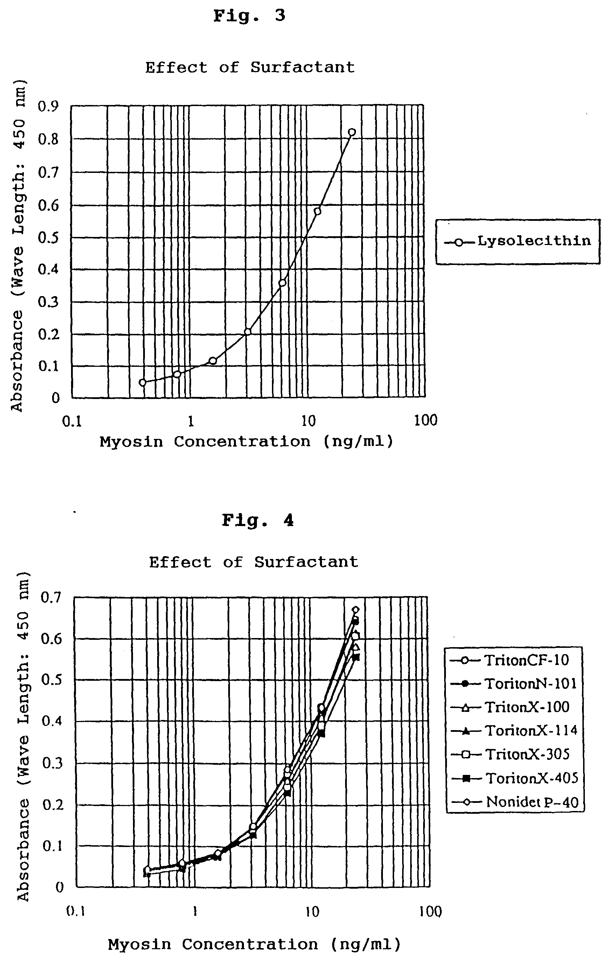

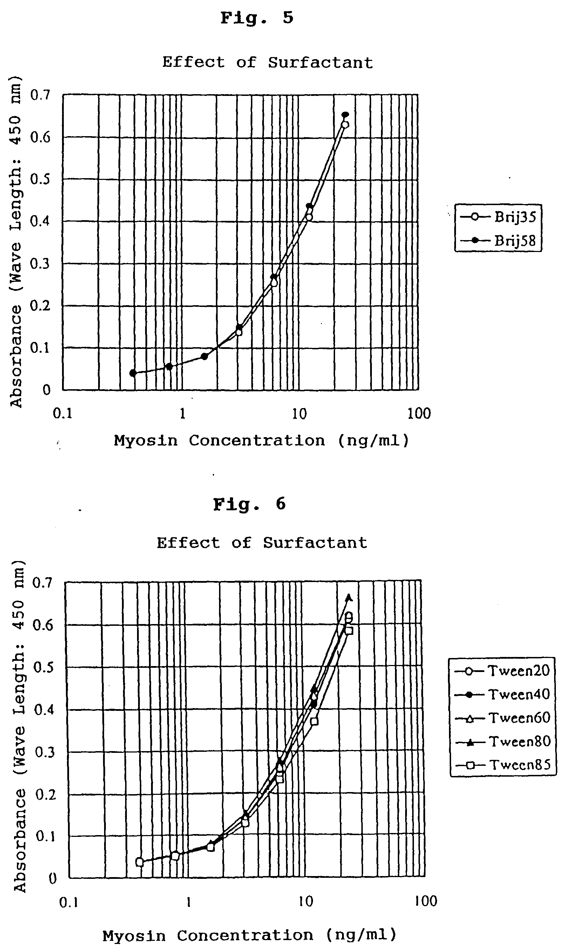

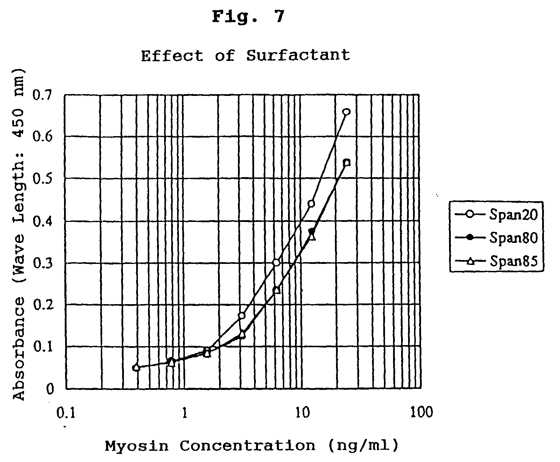

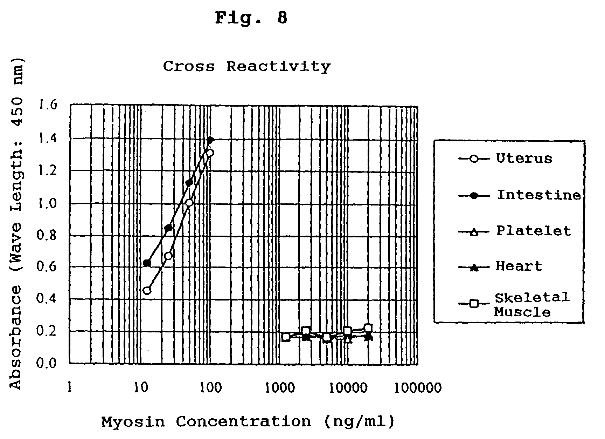

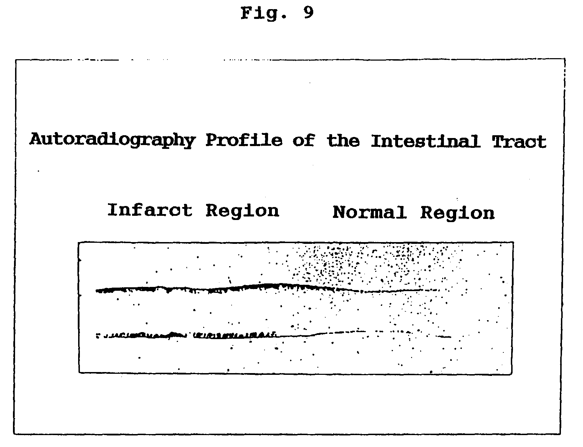

Fig. 1 shows the standard curve. Fig. 2 shows the changes in absorbance for various concentrations of Tween 20 with respect to myosin concentrations when a Tween 20 solution was used as a washing solution for the kit of the present invention. Fig. 3 shows the change in absorbance versus the concentration of myosin when a lysolecithin solution was used as a washing solution for the kit of the present invention. Fig. 4 shows the changes in absorbance versus the concentration of myosin when solutions of Triton CF-10, Triton N-101, Triton X-100, Triton X-114, Triton X-305, Triton X-405, and Nonidet P-40 were respectively used as washing solutions for the kit of the present invention. Fig. 5 shows the changes in absorbance versus the concentration of myosin when solutions of Brij 35 and Brij 58 were respectively used as washing solutions for the kit of the present invention. Fig. 6 shows the changes in absorbance versus the concentration of myosin when solutions of Tween 20, Tween 40, Tween 60, Tween 80, and Tween 85 were respectively used as washing solutions for the kit of the present invention. Fig. 7 shows the changes in absorbance versus the concentration of myosin when solutions of Span 20, Span 80, and Span 85 were respectively used as washing solutions for the kit of the present invention. Fig. 8 shows the results of a cross-reactivity test. Fig. 9 shows the results of an imaging by autoradiography.

### Best Mode for Carrying out the Invention

### (1) Antibodies against human smooth muscle myosin

No particular limitation is imposed on the monoclonal antibody used in the present invention so long as it specifically binds to human smooth muscle myosin, particularly to a heavy chain of human smooth muscle myosin, and there is preferably used a monoclonal antibody having low cross-reactivity to other types of myosin.

Such a monoclonal antibody may be prepared by employment of a known method shown in the below-described Examples (See, e.g., "*Men-eki Seikagaku Kenkyuhou* (*Zoku-Seikagaku Jikken Kouza 5*)," Edition of The Biochemical Society of Japan, pp. 1-88 (1986); Biochemistry, 27, 3807-3811 (1988); Eur. J. Biochem., 179, 79-85 (1989); J. Mol. Biol., 198, 143-157 (1987); J. Biol. Chem., 264, 9734-9737 (1989); J. Biol. Chem., 264, 18272-18275 (1989); J. Biol. Chem., 266, 3768-3773 (1991); and Circulation, 88, 1804-1810 (1993)).

With regard to the monoclonal antibodies used, a monoclonal antibody itself or active fragments of the monoclonal antibody may be used. No particular limitation is imposed on the active fragments; any of a variety of fragments such as F(ab')₂, Fab', or Fab may be used so long as it has characteristics of the monoclonal antibody of the present invention. By use of such active fragments, non-specific adsorption is prevented to thereby minimize measurement errors. When they are administered in blood, the half-life may be reduced and *in vivo* clearance may increase.

Such active fragments may be prepared by employing a known method such as papain-, pepsin-, or tripsin-treatment to a purified monoclonal antibody (See "Men-eki seikagaku kenkyuuhou (Zoku Seikagaku Jikken Kouza 5)," Edition of The Biochemical Society of Japan, pg. 89 (1986)).

The thus-prepared antibody or active fragments may be used as the following antibody reagents or agents for diagnostic imaging.

### (2) Antibody reagents and kits for detection

The antibody reagents of the present invention are used to screen digestive disease through values measured by immuno-assaying human smooth muscle myosin in a sample to be tested. Therefore, the antibody reagents of the present invention must contain at least an antibody against human smooth muscle myosin or an active fragment thereof. Examples of the antibodies and active fragments thereof include those described in the above (1).

Dissolved or lyophilized antibodies or active fragments thereof may be used as the antibody agents, and they may be modified into a form (immobilized antibody, labeled antibody, etc.) suited for an optionally employed assay method.

The antibodies or active fragments thereof may be modified through a customary method. For example, in the case of manufacturing immobilized antibodies, no particular limitation is imposed on the materials usable for supports so long as they bond well to the antibodies or active fragment thereof. Examples of the materials include synthetic organic polymer compounds such as polyvinyl chloride, polystyrene, a styrene-divinylbenzene copolymer, a styrene-maleic anhydride copolymer, Nylon, polyvinyl alcohol, polyacrylamide, polyacrylonitrile, or polypropylene; polysaccharides such as dextran derivatives (e.g., Sephadex), agarose gel (e.g., Sepharose, Biogel), and cellulose (e.g., paper-disk, filter paper); and inorganic polymer compounds such as glass, silica gel, or silicone. Any of these materials may have a functional group such as an amino group, an aminoalkyl group, a carboxyl group, an acyl group, or a hydroxy group.

The shape of the support may be, for example, flat as in the case of a microtiter plate or a disk; granular as in the case of beads; tubular as in the case of a test tube or a tube; fibrous; and membrane-like, and the shape is appropriately selected in accordance with the measurement method.

The antibodies or active fragments thereof may be immobilized onto solid supports through know methods such as a physical adsorption method, an ionic bond method, a covalent bond method, or an entrapping method (See, e.g., "Immobilized Enzymes" edited by Chibata Ichiro, March 20 1975, Kodansha Co., Ltd.).

In the case of preparing labeled antibodies or active fragments thereof, as labels there may be used radioactive isotopes (³²P, ³H, ¹⁴C, ¹²⁵I, etc.); enzymes (β-galactosidase, peroxidase, alkaliphosphatase, etc.); coenzyme-prosthetic groups (FAD, FMN, ATP, biotin, hem, etc.); fluorescent dyes (fluorescein derivative, rhodamine derivative, etc.); and metal particles (gold, silver, platinum, etc.).

The antibodies or active fragments thereof may be labeled in accordance with a known method suitable for the selected labelling agent (See, e.g., "*Zoku-Seikagaku Jikken Kouza 5, Men-eki Seikagaku Kenkyuhou*" Tokyo Kagaku Dojin Co., Ltd., (published in 1986) pp. 102-112).

The kit for detection of the present invention is used in a method in which smooth muscle myosin contained in a sample is to be tested by immuno-assay, and digestive diseases are detected by use of the obtained measurement values. Therefore, the kit is characterized by containing as constitutional reagents at least the above-mentioned antibody reagents and an additional washing solution containing a surfactant.

With regard to the antibody reagents added to the kit for detection of the present invention, from the above-mentioned antibody reagents there may be appropriately selected an antibody reagent having a form suitable for an immuno-assay method conducted by use of the kit.

No particular limitation is imposed on the surfactants added to the washing solutions so long as they have water-solubility, and ampholytic surfactants or nonionic surfactants are particularly preferred. More specifically, examples of the ampholytic surfactants include yolk lysolecithin, and examples of the nonionic surfactants include Tween series (Tween 20, 40, 60, 80, 85, etc.; products of Nakarai Tesuku Co.), Span series (Span 20, 80, 85, 80, etc.; products of Nakarai Tesuku Co.), Brij series (Brij 35, 58, etc.; products of Nakarai Tesuku Co.), and (n)p-t-octylphenyl ether base (Triton CF-10, N-101, X-100, X-114, X-305, X-405, Nonidet P-40, etc., products of Nakarai Tesuku Co.). The appropriate amount of the surfactants added is 0.003% (weight/volume) or more.

Smooth muscle myosin contained in a sample may be measured with higher sensitivity by application of a washing solution containing such surfactants to the kit for detection of the present invention and employment of the obtained kit in the assay.

In addition to the above reagents, there may be used other materials appropriately selected from those (antigen solution, emzyme solution, substrate solution, reaction-terminating liquid, sample-diluting liquid, etc.) generally used in the employed assay method.

No particular limitation is imposed on the assay method which may be employed in conjunction with the kit of the present invention so long as it is already employed for an immuno assay, and there may be used a competitive method, a sandwich method, an agglutination method, or a plot-overlay method.

Detailed measurement in the employed assay method may be conducted through, for example, the methods described in the references cited below.
(a) Edited by Hiroshi Irie "Radioimmuno Assay - Second Series" (Kodansha Co., ltd., published on May 1 1979)
(b) Edited by Eiji Ishikawa "Enzyme Immunoassay" (2nd edition)(Igaku Shoin Co., ltd., published on December 15 1982)
(c) "Clinical Pathology" Special & Extra Edition No. 53 "Immunoassay for Clinical Examination Techniques and Applications" (*Rinsyobyouri Kankoukai*, published in 1983)
(d) "Biotechnology Encyclopedia" (CMC Co., ltd., published on October 9 1986)
(e) Japanese Patent Publication (*kokoku*) No. 6-43998, Japanese Patent Application Laid-Open (*kokai*) Nos. 52-148620 and 54-91296
(f) "Methods in ENZYMOLOGY Vol. 70" (Immunochemical techniques (Part A))
(g) "Methods in ENZYMOLOGY Vol. 73" (Immunochemical techniques (Part B))
(h) "Methods in ENZYMOLOGY Vol. 74" (Immunochemical techniques (Part C))
(i) "Methods in ENZYMOLOGY Vol. 84" (Immunochemical techniques (Part D: Selected Immnoassay))
(j) "Methods in ENZYMOLOGY Vol. 92" (Immunochemical techniques (Part E: Monoclonal Antibodies and General Immnoassay))
   [(f) to (j): published by Academic Press Co.]

The method and the kit of the present invention are further explained below by taking the case of a sandwich method which is a suitable method among assay methods.

### Kit A;

(1) Immobilized first antibody
(2) Second antibody
(3) Labeled anti-immunoglobulin antibody
(4) Concentration-known antigen
(5) Washing solution.

A labeled second antibody may be used instead of the second antibody and labeled anti-immunoglobulin antibody of kit A. In this case, the below-described kit B is exemplified.

### Kit B;

(1) Immobilized first antibody
(2) Labeled second antibody
(3) Concentration-known antigen
(4) Washing solution.

When a biotin-avidin method is used, the below-described kit C is exemplified.

### Kit C;

(1) Immobilized first antibody
(2) Biotinylated second antibody
(3) Labeled avidin
(4) Concentration-known antigen
(5) Washing solution.

The method for measuring smooth muscle myosin in a blood sample such as serum by using the above-described kit is substantially the same as that performed by using other known kits for tests in which a sandwich method is employed. Briefly, there may be used a method which includes reacting an immobilized antibody reagent with a sample to be tested, optionally subjecting to BF-separation, and further reacting a (labeled) antibody reagent (two-step method); or a method which includes allowing an immobilized antibody reagent, a sample to be tested, and a (labeled) antibody reagent to react simultaneously (one-step method). After reaction, in either case, smooth muscle myosin contained in the sample may be detected or quantitatively determined through a method known *per se* in accordance with to the manner of labeling.

Examples of the test samples which serve as objects of measurement include blood collected from patients suspect of suffering digestive diseases or fractions thereof (e.g., serum), and they may be used for measurement by dilution with an appropriate buffer such as PBS as needed.

If the measurement obtained is in excess of the average value of normal individuals, the patient is diagnosed to have a digestive disease with a very high probability, and the patient should be subjected to more detailed examinations, whereas the measurement is equal to or less than the average value of healthy people, it is decided that the patient is unlikely to have a digestive disease.

### (3) Agent for diagnostic imaging

The agent for diagnostic imaging of the present invention is used for performing accurate diagnostic imaging of a site affected by a digestive disease, by causing a radioactive-isotope-labeled monoclonal antibody against human smooth muscle myosin to specifically bind to the affected site. Therefore, the agent must contain at least a radioactive isotope-labeled monoclonal antibody against human smooth muscle myosin.

Any of the monoclonal antibodies described in the above (1) may be used as the monoclonal antibody used in the present invention, and the monoclonal antibodies or active fragments thereof are labeled with radioactive isotopes to serve as the agent for diagnostic imaging of the present invention.

Examples of the radioactive isotopes include iodine-125, iodine-123, iodine-131, indium-111, indium-113m, technetium-99m, gallium-67, lead-203, ruthenium-97, mercury-197, thallium-201, and bismuth-212. The labeling method may be selected from a variety of known methods in accordance with the employed nuclear species.

For example, there may be employed a direct labeling of the antibody or active fragment thereof with radioactive isotopes such as a chloramine T method or a lactoperoxidase method; and a method involving bonding a bifunctional chelating agent to the antibody or active fragments to form a covalent bond and labeling the formed coupling compound with the above nuclear species.

Examples of the bifunctional chelating agents used in the present invention include 1-amino-6,17-dihydroxy-7,10,28,21-tetraoxo-27-(N-acetylhydroxyimino)-6,11,17,22-tetrazaheptaeicosane (despherioxamine), 8-hydroxyquinoline, ethylenediaminetetraacetic acid, diethylenetriaminepentaacetic acid (DTPA), and diaminocyclohexyltetraacetic acid, and these chelating agents and the antibody or active fragments are bonded by a customary method such as a carbodiimide method, an acid anhydride method, or a glutaraldehyde method.

The kit for the diagnosis of the present invention is used to prepare a monoclonal antibody against human smooth muscle myosin or active fragments of the antibody labeled with radioactive isotopes; so as to perform correct diagnostic imaging for a region affected by these diseases by binding the labeled monoclonal antibody or active fragments of the antibody specifically to the region affected by these diseases. Therefore, the kit for the diagnosis must be fabricated so as to enable the preparation of at least a monoclonal antibody against human smooth muscle myosin or active fragments of the antibody labeled with radioactive isotopes. In one specific embodiment, there may be exemplified a kit containing a coupling compound comprising a bifunctional chelating agent and either a monoclonal antibody against human smooth muscle myosin or active fragments of the antibody, as well as a solution of radioactive isotopes.

The diagnostic agent and kit of the present invention may contain, in addition to the above reagents, a chromatographic column to purify nuclear species; a carrier such as a sodium chloride solution or a glucose solution for preparation into a form for administration; other stabilizers; etc.

The diagnostic agent of the present invention is administered to the human body through intravenous injection. Therefore, the diagnostic agent of the present invention is used in a form suitable for intravenous injection by use of the above-mentioned carrier, etc. The diagnostic agent of the present invention is administered in an amount of typically 100 µCi-30 mCi, preferably 500 µCi-3 mCi, depending on the radioactive isotope used for labeling.

The diagnostic imaging using the diagnostic agent of the present invention may be performed by scanning a region such as a region of the patient's abdomen where digestive disorders seem to be generated, and detecting radioactivity attributed to the agent of the present invention to depict the image thereof.

### Examples

The present invention will next be described in detail by way of examples, which should not be construed as limiting the invention.

### Example 1 (Preparation of myosins)

Human uterus smooth muscle myosin, human aorta smooth muscle myosin, human small intestine smooth muscle myosin, human platelet myosin, and human skeletal muscle myosin were provided by Dr. Matsumura, Saga Medical College. Human cardiac muscle myosin was purified according to the method of Yazaki (Circ. Res., 36:208, 1975). For each type of myosin, purity was assessed by SDS-PAGE, and then the concentration of protein was determined according to the method of Lawry (J. Biol. Chem., 193:265-275, 1951) using bovine serum albumin as a standard.

### Example 2 (Preparation of monoclonal antibodies)

### 1) Preparation monoclonal antibody-producing hybridomas

BALB/c mice aged 6- to 8-weeks were immunized intraperitoneally with 25 to 50 µg of human uterus smooth muscle myosin emulsified with Freund's complete adjuvant, 4 to 7 times at 2- to 4-weeks intervals, after which human uterus smooth muscle myosin (10 µg) was administered by intravenous injection.

The spleen of each mouse was removed three days after the final immunization, and the spleen cells and mouse myeloma cells P3×63Ag8U. 1(P3U1) (ATCC CRL-1597) were mixed at a ratio of 10 : 1. An RPMI 1640 solution (1 ml) containing 50% polyethylene glycol was gradually added to pellets obtained by centrifugal separation of the mixture to thereby conduct cell fusion. Subsequently, an RPMI 1640 culture medium was added thereto to adjust the volume to 10 ml and the mixture was centrifuged to thereby obtain pellets, which were suspended in an RPMI 1640 culture medium containing 10% fetal calf serum at the concentration of P3U1 3 × 10⁴ cells/0.1 ml. The suspension was dispensed into a 96-well microplate in an amount of 0.1 ml/well.

After one day an HAT culture medium (0.1 ml) was added to each well, and half of the medium was replaced with fresh HAT medium every 3-4 days.

The culture supernatant was sampled 7-10 days after fusion, dispensed in an amount of 50 µl/well into a 96-well polyvinyl chloride (PVC) plate which had been precoated with human uterus smooth muscle myosin and blocked with 3% gelatin, and allowed to react at room temperature for one hour. After three washings with PBS, a solution containing biotinylated horse anti-mouse IgG (Vector Co.) diluted 500 times with PBS containing 1% bovine serum albumin (BSA) was added in an amount of 50 µl/well and the microplate was allowed to stand at room temperature for one hour. After three washings with PBS, a solution containing peroxidase-avidin D (Vector Co.) diluted 2000 times with PBS containing 1% BSA was added in an amount of 50 µl/well, and the microplate was allowed to stand at room temperature for 15 minutes. After three washings with PBS, 200 µl of a substrate solution (4-aminoantipyrine, 0.25 mg/ml, phenol, 0.25 mg/ml, 0.4 M hydrogen peroxide) was added thereto and the mixture was allowed to develop color at room temperature. The absorbance at 550 nm was measured by use of a microplate photometer, to thereby select hybridomas producing monoclonal antibodies reacting specifically with human uterus smooth muscle myosin.

The thus-selected hybridomas were subjected to cloning by limiting dilution and there were established five strains of hybridoma (1H6, 4E12, 9A12, 9D7, 10G2) against human uterus smooth muscle myosin. The number of specific antibody-positive wells, the number of growth wells, and the total number of wells are shown in Table 1. Of the selected hybridomas, hybridoma 1H6 and hybridoma 4E12 were deposited with National Institute of Bioscience and Human-Technology, Agency of Industrial Science and Technology (1-3, Higashi 1-chome, Tsukuba-shi, Ibaraki-ken 305, JAPAN) as SMHMW1H6 and SMHMW4E12 on October 13, 1994 under Budapest Treaty, and were assigned accession numbers FERM BP-4829 and FERM BP-4830 on October 13, 1994.

**Table 1**

| Wells which indicated positive to specific antibody | Wells which indicated growth | Total No. of wells |
|---|---|---|
| 11 | 653 | 940 |

### 2) Preparation and purification of monoclonal antibodies

Next, cells of each hybridoma established were cultured and then intraperitoneally administered to mice previously given pristane, in number of 3 × 10⁶ per mouse. About two weeks after the administration of hybridomas, 5 ml of ascites was collected from each mouse. The ascites was mixed with an equal volume of 1.5 M glycine-hydrochloric acid buffer (pH 8.9) containing 3 M sodium chloride, and was passed through a Protein A Sepharose CL-4B column (Pharmacia Co.) equilibrated with the same buffer as above. After the column was washed with a sufficient amount of the same glycine-hydrochloric acid buffer, the antibody was eluted with 0.1 M citrate buffer (pH 6.0). The eluate was dialized against PBS, and purity was confirmed by SDS-polyacrylamide gel electrophoresis (SDS-PAGE) to thereby obtain a purified monoclonal antibody.

### Example 3 (Properties of the monoclonal antibodies)

### 1) Isotype

The culture supernatant of each hybridoma was dispensed into a 96-well PVC plate which had been precoated with human uterus smooth muscle myosin and blocked with 3% gelatin, and the isotype of antibodies was determined with MonoAb-ID EIA kit (Zymed Co.).

**Table 2**

| | | | | | |
|---|---|---|---|---|---|
| Hybridoma | 1H6 | 4E12 | 9A12 | 9D7 | 10G2 |
| Isotype | IgG1/κ | IgG1/κ | IgG1/κ | IgG1/κ | IgG1/κ |

### 2) Specificity analysis by Western blotting

The specificities of monoclonal antibodies were analyzed by Western blotting.

Human uterus smooth muscle myosin (1 mg/ml) was mixed with an equal volume of a reducing solution and the mixture was heated at 100°C for five minutes. SDS-PAGE was performed with a mini-gel electrophoresis apparatus (Marysal Co.) using a 10% separated gel and a 5% concentrated gel at 10 mV for approximately three hours. Blotting was performed with a blotting apparatus for mini-gel (Marysal Co.) at 37 V for about 18 hours to transcribe protein to a nitrocellulose membrane, which was subsequently cut along the migration lines into strips. Some of them were treated with Amido Black to stain protein and the remainder were blocked with 3% gelatin and then reacted with a culture supernatant of each hybridoma at room temperature for one hour.

Subsequently, the reaction mixture was twice washed for 10 minutes with 20 mM Tris-500 mM NaCl (pH 7.5) buffer containing 0.05% Tween 20(T-TBS) and reacted with 500 fold-diluted biotinylated horse anti-mouse IgG (Vector Co.) at room temperature for one hour. The reaction mixture was then washed twice with T-TBS for 10 minutes and reacted with 2000 times-diluted peroxidase-avidin D (Vector Co.) at room temperature for 15 minutes. The reaction mixture was then washed twice with T-TBS for 10 minutes, treated with a color development solution containing 30 mg of HRP color development reagent (Bio-rad Co.), 10 ml of methanol, 50 ml of TBS, and 30 µl of a 30% hydrogen peroxide, and then washed with distilled water.

Protein-staining with Amido Black resulted in five observed bands, i.e., 200K (uterus smooth muscle myosin heavy chain), 140K (fragment of uterus smooth muscle myosin heavy chain), 70K (fragment of uterus smooth muscle myosin heavy chain), 20K (uterus smooth muscle myosin light chain), and 17K (uterus smooth muscle myosin light chain). It was confirmed that all antibodies reacted with a human uterus smooth muscle myosin heavy chain and did not react with a light chain thereof.

### Example 4 (Detection kit by use of a sandwich method)

### 1) Preparation of biotinylated antibodies

Each of the above monoclonal antibodies was dialyzed against a 0.1 M sodium hydrogencarbonate solution, and the dialyzed solution was concentrated to 2 mg/ml by use of a Centriflow (Amicon Co.). Biotin (Long-arm) NHS reagent (Vector Co.) was dissolved in dimethylformamide to a concentration of 10 mg/ml, of which 20 µl was mixed with the above antibody solution (1 ml). The mixture was allowed to react at room temperature for two hours. After addition of ethanolamine (5 µl) to terminate reaction, the mixture was twice dialyzed against PBS, to thereby obtain a biotinylated antibody. The biotinylated antibody was diluted with 1% BSA-containing PBS to 1 µg/ml, to thereby obtain a solution of the biotinylated antibody.

### 2) Preparation of immobilized antibodies

An anti-smooth muscle myosin monoclonal antibody (4E12) was diluted with PBS to 10 µg/ml, dispensed into a 96-well plate (H type: Sumitomo Bakelite Co., Ltd.) in an amount of 50 µl/well, and the microplate allowed to stand at 4°C for one night. After the antibody was washed three times with PBS containing 0.05% Tween 20, 0.5% skim milk was dispensed thereto in an amount of 300 µl/well and the plate was allowed to stand at room temperature for one hour. The skim milk solution was removed to thereby obtain an immobilized antibody reagent.

### 3) Preparation of other reagents and preparation of kits

* Standard solutions of smooth muscle myosin;
   To prepare the standard solutions, human aortic smooth muscle myosin was diluted with PBS containing 1% BSA to attain a concentration of 25, 12.5, 6.25, 3.125, 1.563, 0.781, or 0.391 ng/ml.
* Washing solution;
   To prepare the solution, Tween 20 was dissolved in PBS so as to attain a concentration of 0.05% (w/v).
* A solution of enzyme-labeled avidin;
   To prepare the solution, peroxidase-avidin D (A-2004: Vector Co.) was diluted 5000 times with PBS containing 1% BSA.
* A substrate solution;
   To prepare the solution, 3,3',5,5'-tetramethylbenzidine dihydrochloride (TMBZ) and aqueous hydrogen peroxide were dissolved in 0.2 M citrate buffer (pH 3.8) to attain respective concentrations of 0.3 mM and 0.005% (w/w).
* Enzyme reaction stopping solution;
   1N sulfuric acid was used.

The above reagents were accommodated in a single kit, to thereby prepare a detection kit of the present invention.

### Example 5

### 1) Standard curve

To each of well containing an immobilized antibody reagent, 1% BSA-containing PBS was dispensed in an amount of 100 µl, and then a standard solution of smooth muscle myosin was added thereto in an amount of 50 µl. The mixture was stirred and subsequently allowed to stand at room temperature for four hours. After washing three times with a washing solution, a solution of biotinylated antibody (1H6) was added in an amount of 50 µl and the mixture was allowed to stand at room temperature for 30 minutes. The wells were washed three times with a washing solution, and an enzyme-labeled avidin solution was added in an amount of 50 µl. The mixture was allowed to stand at room temperature for 15 minutes. After washing three times with a washing solution, a substrate solution was added in an amount of 100 µl and the mixture was allowed to stand at room temperature for 10 minutes so as to develop color. The enzyme reaction stopping solution was added in an amount of 100 µl to stop reaction, and the absorbance at 450 nm was measured with a microplate photometer. The standard curve obtained is shown in Fig. 1.

### 2) Effect of surfactant

In connection with the procedure of the above-described 1), the amount of Tween 20 added into the washing solution was investigated. As shown in Fig. 2, it was found that the sensitivity of measurement can be significantly enhanced at concentrations of 0.003% (weight/volume) or more. The effect of various surfactants other than Tween 20 was also investigated and compared with that of Tween 20. Briefly, each of the surfactants was added to the washing solution so as to adjust the concentration to 0.05% (weight/volume), to thereby obtain standard curves. As shown in Figs. 3 to 7, it was found that addition of ampholytic surfactants or nonionic surfactants provides remarkable effects as comparable to the effect of Tween 20.

### 3) Reproducibility

In accordance with the procedure of the above-described 1), simultaneous reproducibility and reproducibility between measurement days were investigated by use of 3 kinds of specimens. As is apparent from Tables 3 and 4, excellent reproducibility was obtained with a CV value of within 10% in both cases.

**Table 3**

| Simultaneous reproducibility | | | | | |
|---|---|---|---|---|---|
| | | Measurements ng/ml | Average ng/ml | SD | CV % |
| A | 1 | 1.4 | 1.3 | 0.1 | 6.5 |
| | 2 | 1.3 | | | |
| | 3 | 1.2 | | | |
| | 4 | 1.3 | | | |
| | 5 | 1.2 | | | |
| B | 1 | 4.9 | 5.2 | 0.2 | 4.6 |
| | 2 | 5.0 | | | |
| | 3 | 5.2 | | | |
| | 4 | 5.5 | | | |
| | 5 | 5.3 | | | |
| C | 1 | 16.0 | 15.9 | 0.2 | 1.1 |
| | 2 | 15.8 | | | |
| | 3 | 16.2 | | | |
| | 4 | 15.8 | | | |
| | 5 | 15.8 | | | |

**Table 4**

| Reproducibility between measurement days | | | | | |
|---|---|---|---|---|---|
| | | Measurements ng/ml | Average ng/ml | SD | CV % |
| A | 1 | 1.1 | 1.1 | 0.1 | 4.9 |
| | 2 | 1.1 | | | |
| | 3 | 1.2 | | | |
| | 4 | 1.2 | | | |
| | 5 | 1.1 | | | |
| B | 1 | 5.0 | 5.2 | 0.2 | 4.6 |
| | 2 | 5.1 | | | |
| | 3 | 5.6 | | | |
| | 4 | 5.3 | | | |
| | 5 | 5.0 | | | |
| C | 1 | 14.0 | 15.8 | 1.1 | 6.9 |
| | 2 | 15.9 | | | |
| | 3 | 16.8 | | | |
| | 4 | 15.5 | | | |
| | 5 | 16.6 | | | |

### 4) Cross-reactivity

In accordance with the procedure described in 1), cross-reactivity to various types of myosins was investigated. As shown in Fig. 8, it was confirmed that the antibodies react with uterus smooth muscle myosin and small intestine smooth muscle myosin almost equally, whereas the antibodies exhibited almost no reactivity with skeletal muscle myosin, heart muscle myosin, and platelet myosin (non-muscle type myosin).

### 5) Measurement of smooth muscle myosin in a model case

By use of a group of Wistar rats, each of the rats was subjected to ventrotomy under anesthesia with pentobarbital. The mesenteric artery was ligated, and released at 2 hours after the ligation to cause infarction in the intestine. The concentration of smooth muscle myosin in blood was measured in accordance with the procedure of the above-described 1) at time points of 15 minutes, 30 minutes, 1 hour, 2 hours, and 4 hours after release of ligation.

The concentration of smooth muscle myosin in blood increased 30 minutes after release of ligation and the value reached a peak one hour after release of ligation (30 minutes after: 0.553 ± 0.012 ng/ml, one hour after: 2.334 ± 0.02 ng/ml).

### 6) Measurement of serum of healthy subjects

In accordance with the procedure of the above-described 1), the concentration of smooth muscle myosin was measured in 75 samples of serum from healthy subjects, to thereby obtain an average of 0.9 ng/ml and a standard deviation of 0.9 ng/ml.

### 7) Measurement of serum of patients

In accordance with the procedure of the above-described 1), the concentration of smooth muscle myosin was measured for patients suffering digestive diseases before and after surgery. The changes of the value are shown in Table 5. As is apparent from Table 5, the values were high before surgery and decreased to a normal level after surgery. Thus, the results well reflected the effect of the treatment.

**Table 5**

| Specimen | Disease | Date of blood collection | Measurements (ng/ml) |
|---|---|---|---|
| T.S | Intussusception | | |
| | (pre-surgery) | 94.3.23 | 22.2 |
| | (5 days after) | 94.3.28 | 1.1 |
| D.K | Enterostasis | | |
| | (pre-surgery) | 93.10.21 | 14.8 |
| | (5 days after) | 93.10.25 | 0.8 |

### Example 6

### 1) Labeling of monoclonal antibodies

A 1 mg/ml solution of anti-smooth muscle myosin monoclonal antibody (9D7) (20 µl) and Na¹²⁵I (3.7 MBq) were mixed and a 0.3% solution of chloramine T (5 µl) was further mixed for just 30 seconds with a vortex. To the mixture, a 0.5% solution of sodium pyrosulfite (10 µl), a 1% solution of potassium iodide (5 µl), and a 1% BSA solution (5 µl) were successively added and mixed. The reaction mixture was passed through a PD10 column (Pharmacia Co.) equilibrated with a 1% solution of BSA, then eluted with a 1% solution of BSA, and fractionated by 1 ml. The fractions were measured with an auto-gamma-counter and the counts of them showed two peaks. Fractions attributed to the first peak were collected to obtain a ¹²⁵I anti-smooth muscle myosin monoclonal antibody.

### 2) Diagnostic imaging of an ischemic digestive disease in a model case

The procedure of Example 5 was repeated to cause infarction of the intestine of rats. To each rat, a ¹²⁵I anti-smooth muscle myosin monoclonal antibody (1.85 MBq) was intravenously injected after release of ligation. The rats were sacrificed after 1.5 hours, 6 hours, and 24 hours for removing organs. The ¹²⁵I counts/g of tissue were compared and autoradiographic imaging was conducted.

As shown in Fig. 6, the counts at the infarction parts were already higher than those of normal area after 1.5 hours, and accumulation of ¹²⁵I corresponding to and in agreement with the infarction area was observed in autoradiography.

**Table 6**

| Organs | %count/weight(g) | | |
|---|---|---|---|
| | 1.5 hr | 6 hr | 24 hr |
| Blood | 6.715 | 3.249 | 1.882 |
| Heart | 0.954 | 0.527 | 0.369 |
| Lung | 2.543 | 1.274 | 0.713 |
| Liver | 2.135 | 1.127 | 0.547 |
| Lien | 1.344 | 0.777 | 0.349 |
| Kidney | 1.685 | 1.0 | 0.575 |
| Uterus | 0.333 | 0.347 | 0.779 |
| Aorta | 1.094 | 1.266 | 1.296 |
| Normal intestine | 0.24 | 0.315 | 0.427 |
| Necrotized intestine | 0.968 | 1.339 | 1.497 |
| Muscle | 0.113 | 0.076 | 0.087 |
| Bone | 0.468 | 0.277 | 0.223 |

### Example 7

Hybridoma 8B8 which produces a monoclonal antibody specifically reacting to human small intestine smooth muscle myosin was established with a method similar to that described in 1) of Example 2 through use of human small intestine smooth muscle myosin instead of human uterus smooth muscle myosin. This hybridoma was internationally deposited with National Institute of Bioscience and Human-Technology, Agency of Industrial Science and Technology (1-3, Higashi 1-chome, Tsukuba-shi, Ibaraki-ken 305, JAPAN) as IMH8B8 on March 5, 1996 based on Budapest Treaty, and was allotted an accession number of FERM BP-5444 on March 5, 1996.

The monoclonal antibody produced from this hybridoma was purified by the same method as 2) of Example 2, and properties of the monoclonal antibody were examined in a manner similar to that as described in Example 3. The results are as follows.
1) Isotype : IgG2b/κ
2) Reaction specificity : Reactive with smooth muscle heavy chains and not reactive with light chains.
3) Cross-reactivity : Reactive with small intestine smooth muscle myosin, uterus smooth muscle myosin, and aortic smooth muscle myosin and not reactive with skeletal muscle myosin, heart muscle myosin, or platelet myosin (non-muscle type myosin).
4) Species specificity : Reactive with rat smooth muscle myosin and human smooth muscle myosin.

Next, the monoclonal antibody (8B8) was labeled by the same method as that of 1) of Example 6, and there was performed diagnostic imaging for damaged parts of intestine in a model experiment using rats in accordance with a method described in 2) of Example 6. The results show that accumulation of ¹²⁵I in accordance with the damaged parts of intestine is observed.

### Industrial Applicability

The present inventors have first found that digestive diseases can be biologically diagnosed by the measurement of smooth muscle myosin in a test sample, and that accurate diagnostic imaging of the affected site can be performed by causing a radioactive-isotope-labeled monoclonal antibody against human smooth muscle myosin or active fragments thereof to be specifically bound to or concentrated in the region affected by the digestive disease.

Therefore, the antibody reagents and diagnostic imaging agents of the present invention are useful for diagnostic imaging of digestive diseases including ischemic digestive diseases such as ischemic colitis and enterostasis and ulcerative digestive diseases such as ulcerative colitis. Use of them permits an emergency test to be performed and the identification of the affected site.

## Claims

1. An antibody reagent for detecting a digestive disease, which reagent comprises an antibody against human smooth muscle myosin or active fragments thereof.

2. The antibody reagent according to claim 1, wherein the antibody is a monoclonal antibody.

3. The antibody reagent according to claim 1 or 2, wherein the digestive disease is an ischemic digestive disease or an ulcerative digestive disease.

4. A kit for detecting a digestive disease, which kit comprises an antibody reagent as described in any one of claims 1 through 3.

5. The kit according to claim 4, wherein the digestive disease is an ischemic digestive disease or an ulcerative digestive disease.

6. A method for screening a digestive disease on the basis of measured values obtained by measuring smooth muscle myosin in a sample by immunoassay.

7. The method according to claim 6, wherein the digestive disease is an ischemic digestive disease or an ulcerative digestive disease.

8. An image-diagnostic agent for a digestive disease, which agent comprises a monoclonal antibody against human smooth muscle myosin or active fragments of the antibody labeled with a radioactive isotope.

9. The image-diagnostic agent according to claim 8, wherein the digestive disease is an ischemic digestive disease or an ulcerative digestive disease.

10. A kit for the image diagnosis of a digestive disease, comprising a conjugate formed of a bifunctional chelating agent and a monoclonal antibody against human smooth muscle myosin or active fragments of the antibody, as well as a solution of a radioactive isotope.

11. The kit according to claim 10, wherein the digestive disease is an ischemic digestive disease or an ulcerative digestive disease.

12. A method of diagnostic imaging for a digestive disease, which method makes use of a diagnostic agent as described in claim 8 or 9 or a kit as described in claim 10 or 11.

13. The method according to claim 12 which is used for identifying the site of disease in a patient suffering a digestive disease.
